# EUROPEAN PATENT APPLICATION

(11) **EP 2 975 383 A1**
(43) Date of publication of application: **20.01.2016**
(21) Application number: 14176941.4
(22) Date of filing: 14.07.2014
(51) Int. Cl.: G01N 21/64, G01N 33/49, G01N 21/82

(54) **Method and device for analyzing a fluid medium such as a blood sample**

(71) Applicant: Johann Wolfgang Goethe-Universität, Frankfurt am Main, 60323 Frankfurt am Main (DE); Fraunhofer Gesellschaft zur Förderung der angewandten Forschung e.V., 80686 München (DE)
(72) Inventor: Meyer dos Santos, Sascha, 60528 Frankfurt (DE); Harder, Sebastian, 60599 Frankfurt (DE)
(74) Representative: Lucke, Andreas

(57) **Abstract**

Provided is a method for analyzing a polymerization reaction in a sample. The method comprises the steps of providing for fluorescent particles in the sample, exciting the fluorescence of the fluorescent particles, disturbing the sample for making the fluorescent particles move within the sample, taking images of the fluorescent particles within the sample, determining, for each of at least some of the images taken, a measure being indicative of the movement of the particles within the time period during which the respective image has been taken, and analyzing the polymerization reaction based on the determined measure.

## Description

### FIELD OF THE INVENTION

The present invention is directed at providing a system and method for analyzing a fluid medium. In particular, the present invention is directed at providing a system and method for analyzing a blood sample with regard to coagulation time and a system and method for simultaneously analyzing a blood sample with regard to thrombin generation and coagulation time.

### RELATED PRIOR ART

The coagulation or clotting of blood is a necessary process for stopping internal and external bleeding. In the case of coagulation under physiological conditions, two sub-processes are normally involved. One sub-process is based on thrombocytes, which normally initiate the clotting cascade. If a blood vessel is damaged, the thrombocytes typically adhere to the vessel opening, stick to one another and thus produce the initial wound closure. The second process concerns what is known as plasma clotting, in which the closure, which is still loose, is reinforced by the formation of fibrin threads in order to form a blood clot, also referred to as a thrombus.

Although the ability to coagulate is a vital property for limiting blood loss in the event of injury, it also poses risks for humans. The best-known example for this is what is known as thrombosis, in which a thrombus forms in a vessel and blocks it. Thromboses are often produced in veins, specifically the deep leg veins. If the thrombus detaches, it may reach the pulmonary artery, for example via the inferior vena cava and the right atrium of the heart, and may block said artery, which may lead to a pulmonary embolism. A frequent cause of thromboses is an illness-related rise in the coagulation ability of the blood, which can be counteracted using coagulation-inhibiting drugs, for example Heparin or Argatroban. For diagnosis of the increased tendency to coagulation or in order to set the correct dose of the coagulation inhibitor, the coagulation ability of the blood must be tested, for example by measuring the coagulation time of a blood sample in an apparatus suitable for this purpose. The correct dosage of coagulation-inhibiting agents is extremely critical since an excessive lowering of the tendency to coagulation in turn entails the risk of uncontrolled bleeding, even in the case of relatively minor injuries.

A particularly reliable and precise monitoring of the coagulation ability of the blood is also necessary prior to surgical interventions, because it must be ensured that the bleeding of wounds produced unavoidably during the process will also stop reliably. Another important application is emergency medicine. If, for example in the event of an injury, internal bleeding is suspected, it is extremely important to determine whether the blood is sufficiently capable of coagulation. For example, the injured person for unknown reasons may regularly take coagulation-inhibiting drugs, which would then cause increased internal bleeding. For the emergency doctor, it would therefore be important to be able to test quickly and reliably the ability of the blood to coagulate as a routine measure in order to be able to initiate suitable countermeasures where necessary.

In the art, several methods for determining the blood coagulation time are known.

EP 2669678 A1 describes a method for determining the blood coagulation time based on calculating an indicator characterizing a variation of particle speed from an acquired image of a particle-containing liquid in a fluid chamber. In particular, EP 2669678 A1 proposes determining the variation of the speed by calculating a correlation between images.

GB 2399876 A describes a method for determining the blood coagulation time by detecting the polarization of light emitted from fluorescent particles in a blood sample, the polarization being used to determine whether the particles can move freely through the blood sample thus being indicative of coagulation.

EP 0160389 A1 describes a method for determining the blood coagulation time by adding fluorescent latex particles of different sizes to a whole blood sample.

CN 101308149 A describes a method for determining the blood coagulation time by moving a steel ball in a blood sample wherein the moving steel ball changes an intensity of a light beam guided through the blood sample.

US 2014064595 A1 describes a method for determining the blood coagulation time by adding fluorescent microspheres to a blood sample and analyzing the correlation between images of the fluorescent particles.

### SUMMARY OF THE INVENTION

Although a large number of devices for analyzing a polymerization reaction in a sample such as determining the coagulation time of blood are known, there is still a need for improvements. An advantageous method or apparatus combines a number of properties, including good reproducibility of the measured results, simple operation, a quick analysis time, and a reasonable equipment cost. The object of the invention is to provide methods and apparatuses exhibiting such properties.

A method for analyzing a polymerization reaction in a sample which provides advantages in all these aspects is defined in claim 1. A corresponding apparatus is defined in claim 8. In addition, a method for measuring thrombin generation that is suitable to be combined with the method for analyzing a polymerization reaction in a sample in a multiplex assay and a corresponding system are defined in claims 13 to 15.

In accordance with the invention, the method for analyzing a polymerization reaction in a sample comprises the steps of:
- providing for fluorescent particles in the sample;
- exciting the fluorescence of the fluorescent particles;
- disturbing the sample for making the fluorescent particles move within the sample;
- taking images of the fluorescent particles within the sample;
- determining, for each of at least some of the images taken, a measure being indicative of the movement of the particles within the time period during which the respective image has been taken; and
- analyzing the polymerization reaction based on the determined measure.

A measure that is indicative of a movement of particles within a time period during which an image of the particles has been taken is to be interpreted as a measure based on properties of an over-exposed image, imaging the moving particles in a blurred fashion. As long as the particles move rather freely within the disturbed sample, an over-exposed image of the particles will not exhibit sharply imaged particles placed on fixed positions but rather blurry particle trajectories, i.e., trajectories along which the particle move during the exposure time.

As the polymerization process proceeds, the particles slow down and the trajectories of the particles in the images become shorter, sharper and brighter, as more light incidents on a smaller region of the imaging sensor. In other words, the blurry, bright regions decrease in size while increasing in sharpness and brightness. When the blood has clotted, each particle is prevented from moving through the sample and the particles are imaged without any blur or distortions. For example, fluorescent microspheres are imaged as bright circles with sharp edges. The inventors have found that analyzing over-exposed images instead of calculating the correlation between two images requires fewer resources and provides better results in view of analyzing the polymerization reaction occurring in the sample.

In an advantageous embodiment, analyzing the polymerization reaction in the sample comprises determining a coagulation time in a blood sample and the step of analyzing the polymerization reaction based on the determined measure includes determining the coagulation time based on a recording time of an image for which the measure reaches a predetermined threshold.

It should be noted that in this context the term "blood sample" is understood to mean any liquid in which blood is contained, but that further components may also be present, in particular calcium chloride for recalcification of the blood, and possibly a coagulation stimulator, such as kaolin. Although whole blood is preferably examined within the scope of the invention, some components of the blood may optimally also be removed in the sample.

Furthermore, although the following description focusses on analyzing a blood sample, it is clear to the skilled person that any substance which is subject to a polymerization process can be analyzed using the disclosed methods and apparatus. Accordingly, the term "blood sample" or "blood" as used in the following could be replaced by the term "substance that is subject to a polymerization process" or "sample" with blood or blood plasma being but one substance that is susceptible to the disclosed methods and apparatus.

Of course, coagulation as such is a process, and therefore there is not strictly speaking a clearly defined "moment" of coagulation, where, for example, particle movement stops abruptly. Instead, a criterion has to be defined within the scope of the invention, on the basis of which the "moment of coagulation" is established. Provided this criterion is applied consistently, a reproducibility of the measured values of the coagulation time is provided that is found remarkable by the inventors. These measured values can then be associated by means of comparison or reference measurements with the clinically relevant dose range in anticoagulation therapy.

The exposure time that is required for making the movement of the particles visible in a single image depends inter alia on the speed at which the particles move within the un-clotted blood sample. If the speed at which the particles move within the un-clotted blood sample cannot be determined or estimated in advance, the method may comprise a further step of setting the exposure time on basis of one or more calibration images, wherein the calibration images are imaged at different exposure times. An exposure time may be selected, for which the fluorescent particles within the image meet a predetermined blur criterion, the blur criterion being related to blur that is caused by moving objects imaged by an over exposed image. For example, the blur criterion may be that the size of the imaged particles, which are shown in the image as bright regions, is equal to or above a multiple of the real particle size.

As an alternative, the blur criterion may be that a binary image of the fluorescent particles exhibits a mean brightness that is within a predetermined brightness range or above or below a predetermined brightness value. For example, when the fluorescent particles move freely within the blood sample, the light by the fluorescent particles is not focused but spread over the light sensitive surface of the imaging sensor and thus, an image of moving fluorescent particles comprises larger regions with reduced brightness and blurry edges compared to an image of non-moving particles, which comprises smaller regions with higher brightness and sharp edges. Hence, a binary image may exhibit none or only a few bright regions when the fluorescent particles move freely within the blood sample while exhibiting bright regions for each imaged particle, thereby increasing mean image brightness which may be defined, for example, as the percentage of bright pixels in the overall image. The blur criterion could thus be that the difference between mean brightness of an image of moving particles and an image of non-moving particles is above a predetermined threshold.

In view of adequate resolution, the exposure time during which the respective image has been taken may be chosen to be below a predetermined fraction of an expected coagulation time of the blood sample. Preferably, the exposure time is between 1 ms and 10 s and even more preferably between 10 ms and 70 ms.

In an advantageous embodiment, disturbing the blood sample for making the fluorescent particles move within the blood sample comprises the steps of generating surface waves and coupling the surface waves into the blood sample. For example, generating surface acoustic waves preferably comprises providing a piezoelectric substrate on which an electrode structure is formed and an alternating current source, which is connected to the electrode structure. The piezoelectric substrate may consist, for example, of lithium niobate (LiNbO3). In other words, generating surface waves may comprise providing a device with a surface acoustic wave (SAW) chip, wherein the blood sample is placed on the surface of said device.

The inventors found it to be particularly useful to adjust the input power of the surface acoustic waves (that directly control the velocity of the particles within the blood sample) so that typical coagulation times in untreated blood are 117.3 ± 1.8 seconds which fits to a typical exposure time of 38 ms.

In an advantageous embodiment, the image is taken by an image sensor, using a charge coupled device (CCD) or a complementary metal-oxide-semiconductor (CMOS) image sensor, wherein the image sensor is exposed to the fluorescence of the fluorescent particles while the respective image is being taken. The image sensor may be part of a camera. The camera may comprise a shutter that opens up for letting light incident on the image sensor at the beginning of the exposure time and closes at the end of the exposure time, thereby exposing the image sensor to the fluorescence during the exposure time.

In an advantageous embodiment, the method further comprises the step of converting the image to a binary image comprising only dark and/or bright pixels, wherein the conversion is based on a predetermined conversion threshold. As discussed above, a binary image is to be understood as a digital image that has only two possible values for each pixel. Typically, the two colors used for a binary image are black and white though any two colors may be used. The conversion threshold may be a greyscale value or intensity. The threshold is chosen so that the light intensity increase in a predetermined area of the imaging sensor due to a moving particle coming to stand-still affects a binary image of the particle, for example by a dark region turning into a bright region. Thus, the threshold is preferably such that light emitted from a non-moving particle incidents on a first area of the imaging sensor with an aggregated intensity per area that is above the threshold while light emitted from a moving particle incidents on a second larger area of the imaging sensor with an aggregated intensity per area that is below the threshold.

In an advantageous embodiment, the method further comprises the step of detecting edges in the image. Edge detection identifies pixels in a digital image at which the image brightness changes sharply, i.e. more than a predetermined value. The identified pixels can then be organized into a set of curved line segments termed edges. The edge detection allows dividing the image into segments which can then be further analyzed as will be detailed below.

In an advantageous embodiment, the method further comprises the step of determining all objects in the image whose size is smaller or greater than a predetermined size threshold or within a predefined size range. For example, a binary image may be analyzed for objects, i.e. pixel sets, with the same color that are surrounded by pixel sets of the other color. Analogously, an image on which edge detection has been performed may be analyzed for pixel sets that are surrounded by an edge. The objects whose size is smaller or greater than a predetermined size threshold or within a predefined size range may be determined, for example, by counting the number of pixels in each pixel set or by measuring the length of one or more object axes.

In an advantageous embodiment, the method further comprises the step of determining all objects to which a roundness measure can be assigned that is above a predetermined roundness threshold, an object with a perfect circular shape having a highest roundness measure and a line object having a lowest or minimal roundness measure.

In an advantageous embodiment, the size threshold is determined based on a specific particle-size distribution of the particles in the blood sample. If the size of the fluorescent particles is not known, the size threshold may be determined by analyzing an image of the non-moving particles or by analyzing an image of the moving particles with an adequately short exposure time. In particular, some of the above stated image analysis functions may be used to determine particles in the image and to determine the size of at least some of the particles, wherein the size threshold may be a mean or maximum value of the determined particle size plus a predetermined safety margin.

In an advantageous embodiment, the roundness threshold is determined based on a specific particle-roundness distribution of the particles in the blood sample. The roundness may be a measure indicative of whether all pixels of an object edge are equidistant to a common center and preferably indicative of a variation of a distance between each object edge pixel and a common center.

In an advantageous embodiment, the measure is a number of detected fluorescent particles in the image. For example, the above indicated size and/or roundness criterion may be used to detect particles and the number of the detected particles may be indicative of the coagulation state of the blood sample. For example, when it is known that 1000 particles are in the sample and the number of detected particles starting by zero and increases to 900, the coagulation time may be determined as a time difference between imaging of the first image where zero particles were detected and imaging of the image where 900 particles were detected

In an advantageous embodiment, the measure is indicative of the mean brightness of the image which may be indicative of the ratio of bright and dark pixels in the image. In particular, a binary image may exhibit only dark pixels when the fluorescent particles move freely within the blood sample as the aggregated light intensity is spread over the imaging sensor and the light intensity per area incident on the imaging sensor may thus not exceed a threshold vale on basis of which the binary image is created. When particle movement decreases, light intensity concentrates on certain regions which are then imaged as bright regions in the image, changing the mean brightness of the image.

In an advantageous embodiment, the particles are microspheres. Microspheres are commercially available in various embodiments with the desired physiochemical properties, such as diameter, fluorescence wavelengths and surface chemistry. For further details, reference is made to the overview article "Microspheres for biomedical applications: preparation of reactive and labelled microspheres" by Reza Arshady, Biomaterials. 1993;14(1):5-15 and "Polymer microbeads in immunology" by V 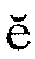 tvicka et al., Biomaterials. 1987 Sep;8(5):341-5. Preferably, the microspheres have a size of less than 10 µm.

In accordance with the invention, an apparatus for analysing a polymerization reaction in a fluid medium which comprises fluorescent particles is provided. The apparatus comprises:
- a sample vessel for receiving a fluid medium, or a receptacle for a sample vessel;
- a light source, the light source being adapted to excite the fluorescence of the fluorescent particles;
- a vibration generating device suitable for disturbing the fluid medium in the sample vessel;
- an imaging device suitable for imaging an image of the fluorescent particles in the fluid medium, wherein the exposure time of the imaging device is above a first predetermined limit and below a second predetermined limit; and
- an image analysis device coupled to the imaging device, the image analysis device being adapted to receive an image from the imaging device and to analyze the image, wherein the analysis is based on an effect which is indicative of the movement of the fluorescent particles during the exposure time, and to determine a polymerization state based on the effect..

It is shall be understood that the sample vessel as such does not necessarily have to be a component of the protected apparatus, but in contrast can be sold for example as a disposable article independent of the apparatus as such. In this case, the apparatus has a receptacle however, that is to say any type of mounting surface or holder for such a sample vessel suitable for use with the apparatus.

The vibration generating device is preferably a device for generating surface acoustic waves. The device for generating surface acoustic waves preferably comprises a piezoelectric substrate, on which an electrode structure is formed, and an alternating current source, which is connected or is connectable to the electrode structure. The piezoelectric substrate consists for example of lithium niobate (LiNbO3).

The electrode structure preferably comprises at least two comb-like electrode arrangements each with a plurality of parallel fingers that are arranged so as to engage with one another at least in part. When an alternating current signal is applied to the two comb-like electrode arrangements, an electric field is generated between the adjacent electrode fingers and the piezoelectric effect leads to a deflection, corresponding to the excitation voltage, of the piezoelectric material and, with suitable frequency of the alternating current signal, to the generation of surface acoustic waves.

In an advantageous embodiment, analyzing a polymerization reaction in a fluid medium comprises determining a coagulation time of blood or plasma and the image analysis device is adapted to determine the coagulation time based on an instant at which the effect meets a predetermined criterion.

A particular advantage of the apparatus is that it can be highly miniaturised. This is true for the equipment set-up itself, which can be kept extremely small and compact and is therefore ideally suitable for a portable device which can also be used outside medical laboratories, for example directly at the hospital bedside, in care establishments, in emergency vehicles or in a patient's domestic environment. A further aspect of miniaturisation concerns the necessary quantity of the blood sample. It has been found that the analysis can be carried out very successfully with use of blood samples that contain merely 5 µl of whole blood, that is to say one drop of blood. The sample vessel therefore preferably has a receiving volume of less than 40 µl, preferably less than 20 µl. The ability to generate meaningful and reproducible results with very small blood sample volumes is advantageous in particular in the case of animal tests carried out on small animals, such as mice, which only have a small quantity of blood.

In an advantageous embodiment, the sample vessel consists of a biocompatible polymer material that constitutes a single user cartridge. In an advantageous embodiment, the device for generating surface waves is adapted to couple the surface acoustic waves into the fluid medium. For, example, the device for generating surface waves comprises a surface acoustic wave, SAW, chip and the sample vessel is placed on the surface of the device for generating surface waves.

In an advantageous embodiment, the image analysis device is preferably adapted to perform one or more of the above discusses image analysis steps. That is, the image analysis device is preferably adapted to:
- convert the image to a binary image based on a predetermined conversion threshold;
- perform edge detection on the image;
- recognize objects that meet a predetermined size criterion; and/or
- recognize objects that meet a predetermined roundness criterion.

As already discussed above, the size criterion may be based on a specific particle-size distribution, the roundness criterion may be based on a specific particle-roundness distribution and/or the criterion which the effect meets may be a number of recognized particles. Thus, in an advantageous embodiment, the image analysis device is further adapted to determine a number of fluorescent particles in the image that exhibit a predetermined size and/or a predetermined roundness and the predetermined criterion is a number of fluorescent particles in the image that exhibit the predetermined size and/or the predetermined roundness.

In an advantageous embodiment, the criterion is indicative of the mean brightness of the image.

In an advantageous embodiment, the particle size may be less than 10 µm.

In accordance with the invention, a method for analyzing a blood sample is provided. The method comprises the steps of:
- adding a thrombin substrate such as Z-Gly-Gly-Arg-AMC-HCl to the blood sample;
- exciting fluorescence in thrombin the thrombin decomposed thrombin substrate; and
- measuring thrombin generation in the blood sample by analyzing the intensity of the detected fluorescence over time.

In an advantageous embodiment, the method further comprises any of the method steps of the above recited method for determining the coagulation time of blood.

This is advantageous in that a method for simultaneously measuring thrombin generation and coagulation time in a blood sample is provided. In an advantageous embodiment, the blood sample comprises whole blood.

In accordance with the invention, a system for simultaneously measuring a coagulation time of and a thrombin generation in a blood sample is provided. The system comprises:
- a sample vessel for receiving a blood sample or a receptacle for a sample vessel;
- a vibration generating device suitable for disturbing the blood sample in the sample vessel;
- a light source suitable for exciting fluorescence of fluorescent particles contained in the sample vessel and for exciting fluorescence of a fluorescent, thrombin decomposed thrombin substrate; and
- an imaging device comprising at least two colour channels for monitoring the fluorescence of the fluorescent particles in a first colour channel and the fluorescence of fluorescent, thrombin decomposed thrombin substrate in a second color channel.

In an advantageous embodiment, the thrombin substrate is Z-Gly-Gly-Arg-AMC-HCl.

### BRIEF DESCRIPTION OF THE FIGURES

Further advantages and features of the invention will become apparent from the following description, in which the invention will be described on the basis of an exemplary embodiment with reference to the accompanying drawings, in which:
- Figure 1: shows a schematic set-up of an apparatus according to the exemplary embodiment,
- Figures 2(a) to (e): show a sequence of schematic images that illustrate the functionality of the apparatus according to the exemplary embodiment,
- Figures 3(a) and (b): show results achieved by the apparatus of the exemplary embodiment and an apparatus based on determining blood coagulation by calculating image correlations; and
- Figure 4: shows a simultaneous measurement of thrombin generation and blood coagulation time.

### DESCRIPTION OF THE PREFERRED EMBODIMENT

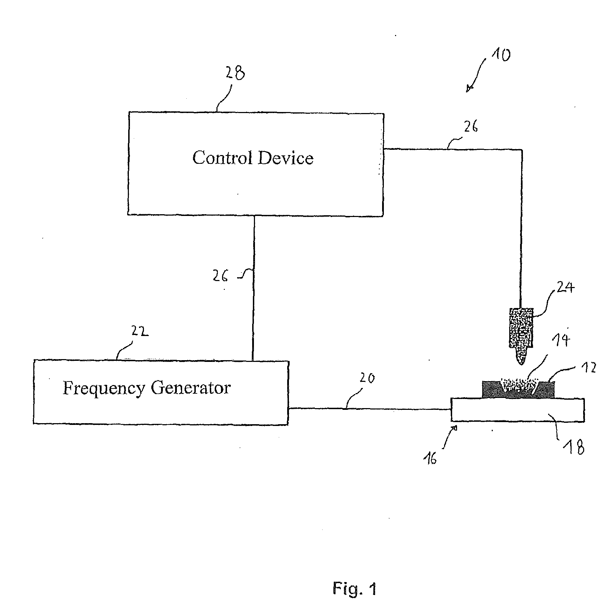

In Figure 1, the set-up of an apparatus 10 for determining the coagulation time of a fluid medium such as a blood sample according to an embodiment of the invention is illustrated schematically. The apparatus 10 comprises a receptacle 12 for receiving a sample vessel 14. The receptacle is located on a device 16 for generating surface acoustic waves, which are also referred to in the literature as SAWs. The device 16 comprises a piezoelectric chip 18, on the upper face of which comb-like electrode arrangements (not shown) are formed, each having a plurality of parallel fingers that engage with one another. The electrode arrangements (not shown) are connected via a line 20 to an alternating current source, more specifically a frequency generator 22.

Above the sample vessel 14, a miniaturised fluorescence microscope 24 is arranged, which is known *per se* and does not need to be explained here in detail. The fluorescence microscope 24 comprises a light source (not shown) for exciting the fluorescence of microspheres (not shown in Figure 1) contained in the sample vessel 14 and also comprises an imaging optics (not shown) which is suitable for imaging an image of the fluorescent microspheres onto an image sensor. Both the fluorescence microscope 24 and the frequency generator 22 are connected via data lines 26 to a control device 28.

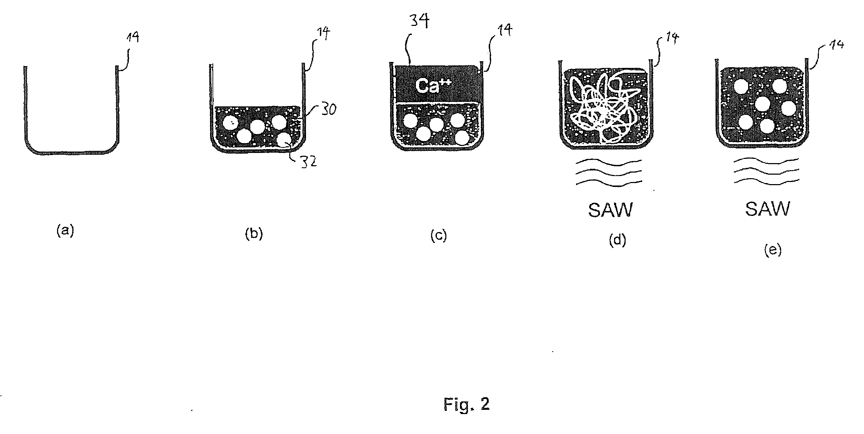

Next, the functionality of the apparatus 10 will be described with reference to Figure 2.

Figure 2(a) shows the empty sample vessel 14 in a schematic illustration.

Figure 2(b) shows the sample vessel 14, which is filled with a blood sample 30 in which biocompatible fluorescent microspheres 32 are contained. Fluorescent microspheres 32 of this type are known from chemical and biological analytics. In the exemplary embodiment shown, they consist of a biocompatible material and have a diameter of approximately 10 µm. In the binary image of Figure 2(b), the fluorescent microspheres are imaged as bright circles.

As is shown in Figure 2(c), calcium chloride is then added and forms Ca++ ions in the solution, which contribute to recalcification and thus initiate clotting. Optionally (not shown), a thrombin substrate such as Z-Gly-Gly-Arg-AMC-HCl can be added to the solution.

With the aid of the device 16, surface acoustic waves are then generated and coupled into the sample liquid, as is illustrated schematically in Figure 2(d). Graphically speaking, the surface acoustic waves coupled into the sample liquid act as a "nano-earthquake" and lead to intense mixing of the blood sample 30 and simultaneously to a rapid movement of the fluorescent microspheres 32, which is illustrated schematically in Figure 2(d). This movement can be monitored with the aid of the fluorescence microscope 24 (see Figure 1). To this end, besides an imaging optics, the fluorescence microscope 24 also contains an image sensor, recording digital images that are transmitted through the data line 26 to the control device 28. The rapid movement is imaged by the trajectories of the microspheres 32. As long as the microspheres 32 move, no bright circles with sharp edges are imaged as shown in Figure 2(c).

In addition, the thrombin generation can be monitored by measuring the fluorescence emitted at 440 nm by the thrombin decomposed Z-Gly-Gly-Arg-AMC-HCl excited with light of a wavelength of 360 nm. Heretofore, the image sensor has two color channels or two changeable color filters, where one color channel or color filter is employed for measuring the thrombin generation while the other color channel or color filter is employed for determining the blood coagulation time.

When coagulation initiates, the mixing of the blood sample and the movement of the microspheres 32 decelerates, in spite of ongoing excitation by the surface acoustic waves. This deceleration or standstill of the movement can be determined automatically by image analysis carried out by the control device 28.

As is further shown in Figure 2(d), the moving particles are no longer imaged by the trajectories of the microspheres 32 but as bright circles. Thus, the deceleration of the microspheres 32 is accompanied by a change in how the microspheres 32 are imaged by the fluorescence microscope 24 as indicated by the transition from Figure 2(d) to Figure 2(e). Hence, the coagulation which leads to the deceleration of the microspheres 32 can be measured by analysing the microsphere images. As shown in Figure 2(e), the microspheres 32 are imaged as bright circles when the clotting of the blood sample forces the microspheres 32 to stand still. Accordingly, the size and roundness of the bright images are valid criteria or effects on basis of which the blood coagulation time can be measured.

In this particular embodiment, the number of bright regions whose size is below 100 pixels and whose roundness is above 0.89 (1.0 being a perfect circle) is counted. A suitable function for determining the roundness is implemented for example in ImageJ (cf. "ImageJ User Guide IJ□ 1.46r" available at http://rsbweb.nih.gov/ij/docs/guide/146-30.html).

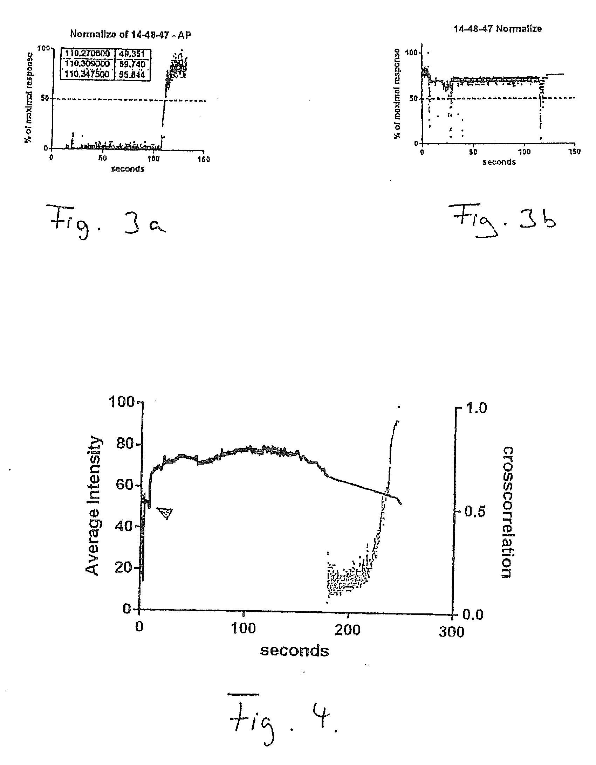

Figure 3(a) shows a measurement of blood coagulation time achieved by the apparatus of the preferred embodiment and Figure 3(b) shows a measurement of the blood coagulation time based on calculating image correlations of consecutive images. Figure 3(a) and Figure 3(b) operate on the same image data. As shown in Figure 3(a), the coagulation time can be precisely determined as the 50% mark is only crossed once. In contrast thereto, Figure 3(b) shows that by calculating the correlation of consecutive images, the 50% mark is crossed three times so that an exact determination of the blood coagulation time is not possible.
Figure 4 shows a simultaneous determination of thrombin generation and blood coagulation time. The measurement is performed using a grayscale image sensor where two fluorescence filters are used. During the first 180 seconds, the thrombin generation, which starts with recalcification at 5 seconds from the start of the curve (indicated by the arrow is monitored).
After 180 seconds, another fluorescence filter is used and the blood coagulation is monitored as described above.

Although Figure 4 relates to a simultaneous determination of thrombin generation and blood coagulation time, it is also possible to measure only the thrombin generation or only the coagulation time.

### LIST OF REFERENCE SIGNS

- 10: apparatus for determining the coagulation time
- 12: receptacle for receiving a sample vessel 14
- 14: sample vessel
- 16: device for generating surface waves
- 18: piezoelectric chip
- 20: line
- 22: frequency generator
- 24: fluorescence microscope
- 26: data lines
- 28: control device
- 30: blood sample
- 32: microspheres
- 34: recalcification reagent

## Claims

1. A method for analyzing a polymerization reaction in a sample, the method comprising the steps of:
providing for fluorescent particles in the sample;
exciting the fluorescence of the fluorescent particles;
disturbing the sample for making the fluorescent particles move within the sample;
taking images of the fluorescent particles within the sample;
determining, for each of at least some of the images taken, a measure being indicative of the movement of the particles within the time period during which the respective image has been taken; and
analyzing the polymerization reaction based on the determined measure.

2. The method of claim 1, wherein the sample is a blood sample and wherein analyzing the polymerization reaction in the sample comprises determining a coagulation time of the blood sample and wherein the step of analyzing the polymerization reaction based on the determined measure includes determining the coagulation time based on a recording time of an image for which the measure reaches a predetermined threshold,
wherein an exposure time during which the respective image has been taken is preferably within 1 ms to 10 s and more preferably within 10 ms to 70 ms.

3. The method of one of the preceding claims, wherein disturbing the sample for making the fluorescent particles move within the sample comprises the steps of generating surface acoustic waves and coupling the surface acoustic waves into the sample, and/or
wherein the image is taken using a charge coupled device, CCD, or a complementary metal-oxide-semiconductor, CMOS, image sensor, wherein the image sensor is exposed to the fluorescence of the particles while the respective image is being taken.

4. The method of one of the preceding claims, further comprising the step of converting the image to a binary image comprising only dark and/or bright pixels based on a predetermined conversion threshold, wherein the measure is preferably indicative of the mean brightness of the image, and/or
further comprising the step of detecting edges in the image.

5. The method of one of the preceding claims, further comprising the step of determining all objects in the image whose size is smaller or greater than a predetermined size threshold or within a predefined size range,
wherein the size threshold or the size range is preferably determined based on a specific particle-size distribution of the particles in the sample.

6. The method of one of the preceding claims, further comprising the step of determining all objects to which a roundness measure can be assigned that is above a predetermined roundness threshold, an object with a perfect circular shape having a highest roundness measure and a line object having a lowest roundness measure,
wherein the roundness threshold is preferably determined based on a specific particle-roundness distribution of the particles in the sample.

7. The method of one of the preceding claims, wherein the measure is a number of detected fluorescent particles in the image, and/or
wherein the particles are microspheres with a size of less than 10 µm.

8. An apparatus (10) for analyzing a polymerization reaction in a fluid medium which comprises fluorescent particles, the apparatus comprising:
a sample vessel (14) for receiving the fluid medium, or a receptacle (12) for a sample vessel (14);
a light source, the light source being adapted to excite the fluorescence of the fluorescent particles;
a vibration generating device suitable for disturbing the fluid medium in the sample vessel;
an imaging device suitable for imaging an image of the fluorescent particles in the fluid medium, wherein the exposure time of the imaging device is above a first predetermined limit and below a second predetermined limit; and
an image analysis device coupled to the imaging device, the image analysis device being adapted to receive an image from the imaging device and to analyze the image, wherein the analysis is based on an effect which is indicative of the movement of the fluorescent particles during the exposure time, and to determine a polymerization state of the fluid medium based on the effect.

9. The apparatus of claim 8, wherein analyzing a polymerization reaction in the fluid medium comprises determining a coagulation time of blood or plasma and wherein the image analysis device is adapted to determine the coagulation time based on an instant at which the effect meets a predetermined criterion, and/or
wherein the vibration generating device is a device for generating surface acoustic waves and coupling the surface acoustic waves into the fluid medium.

10. The apparatus of claim 8 or 9, wherein the image analysis device is further adapted to convert the image to a binary image based on a predetermined conversion threshold and/or to perform edge detection on the image, wherein the criterion is preferably indicative of the mean brightness of the image, and/or
wherein the image analysis device is further adapted to recognize objects that meet a predetermined size criterion, wherein the size criterion is preferably based on a specific particle-size distribution, and/or
wherein the image analysis device is further adapted to recognize objects that meet a predetermined roundness criterion, wherein the roundness criterion is preferably based on a specific particle-roundness distribution,
wherein the criterion is in particular a number of recognized particles.

11. The apparatus of claims 8 or 9, wherein the image analysis device is further adapted to determine a number of fluorescent particles in the image that exhibit a predetermined size and/or a predetermined roundness and the predetermined criterion is a number of fluorescent particles in the image that exhibit the predetermined size and/or the predetermined roundness.

12. The apparatus of one of claims 8 to 11, wherein the particle size is less than 10 µm.

13. A method for analyzing a blood sample, the method comprising the steps of:
adding a thrombin substrate to the blood sample;
exciting fluorescence in the thrombin decomposed thrombin substrate; and
measuring thrombin generation in the blood sample by analyzing the intensity of the detected fluorescence over time.

14. The method according to claim 13, further comprising the steps of any of claims 2 to 7.

15. A system for simultaneously measuring a coagulation time of and a thrombin generation in a blood sample, the system comprising:
a sample vessel (14) for receiving a blood sample or a receptacle (12) for a sample vessel (14);
a vibration generating device suitable for disturbing the blood sample in the sample vessel (14);
a light source suitable for exciting fluorescence of fluorescent particles contained in the sample vessel (14) and for exciting fluorescence of a fluorescent, thrombin decomposed thrombin substrate; and
an imaging device comprising at least two colour channels for monitoring the fluorescence of the fluorescent particles in a first colour channel and the fluorescence of a fluorescent, thrombin decomposed thrombin substrate in a second color channel.
